# EUROPEAN PATENT APPLICATION

(11) **EP 4 603 590 A1**
(43) Date of publication of application: **20.08.2025**
(21) Application number: 24744906.9
(22) Date of filing: 18.01.2024
(51) Int. Cl.: C12P 13/02, C12N 1/20, C12R 1/185, C12R 1/07, C12R 1/85, C12R 1/15

(54) **METHOD FOR OBTAINING HIGH-PURITY SALT OF D-PANTOTHENIC ACID WITH HIGH YIELD**

(30) Priority: 19.01.2023 KR 20230007844
(71) Applicant: CJ CheilJedang Corporation, Seoul 04560 (KR)
(72) Inventor: HONG, Je-Won, Seoul 04560 (KR); KIM, Young-Gon, Seoul 04560 (KR); LIM, Hwayeon, Seoul 04560 (KR)
(74) Representative: Meissner Bolte Partnerschaft mbB
(86) International application number: PCT/KR2024/000908
(87) International publication number: WO 2024/155127

(57) **Abstract**

The present application relates to a method for producing a salt of pantothenic acid, whereby the salt of pantothenic acid can be obtained with high purity without using an anion exchange resin.

## Description

### TECHNICAL FIELD

### Cross-reference with related application(s)

The present application claims benefit of priority to Republic of Korea Patent Application No. 10-2023-0007844, filed on January 19, 2023, the entire contents of which are incorporated herein by reference.

The present application relates to a method of producing a salt of pantothenic acid.

### BACKGROUND ART

Pantothenic acid for food is mainly prepared by synthesis or fermentation. When prepared by fermentation, impurities such as fermentation byproducts should be removed to obtain high-purity calcium D-pantothenate, but the addition of a multi-step purification process has difficulty in reducing the recovery rate of calcium D-pantothenate.

Although patents have been filed for such a method of purifying pantothenic acid, there are problems that the yield of pantothenic acid is low or low-purity pantothenic acid not suitable for food is obtained according to including the multi-step purification process.

Therefore, it is necessary to develop a technology for simultaneously obtaining high-purity pantothenic acid while increasing the yield of pantothenic acid.

### [PRIOR ART DOCUMENT]

(Patent Document 1) US 6013492 A

### DISCLOSURE

### TECHNICAL PROBLEM

An object of the present application is directed to providing a method of producing a salt of pantothenic acid.

### TECHNICAL SOLUTION

An aspect of the present application provides a method of producing a salt of pantothenic acid.

Pantothenic acid is a kind of the vitamin B complex also called vitamin B5. The pantothenic acid may be D-pantothenic acid.

In order to use the salt of pantothenic acid for food, a purification process with a high purity, for example, 98% or more purity, is required. Accordingly, the present application provides a method of producing a high-purity salt of pantothenic acid.

The producing method comprising the steps of:
(a) passing a fermentation liquor which contains pantothenic acid and from which bacterial cell has been removed through a cation exchange resin to obtain an eluent from which impurity has been removed;
(b) adjusting the pH of the eluent obtained in the step (a); and
(c) adding an organic solvent to the pH-adjusted eluent obtained in the step (b).

The producing method may not comprise using of an anion exchange resin.

### Step (a)

The step (a) may be a step of passing a fermentation liquor which contains pantothenic acid and from which bacterial cells have been removed through a cation exchange resin to obtain an eluent from which impurities have been removed.

In this application, the term "fermented product" may refer to a result of enzymatic or metabolic decomposition of an organic substance using a microorganism. For example, the fermented product may comprise a culture itself obtained by culturing a microorganism in a culture medium, or a concentrate, dried product, or freeze-dried product of the culture obtained by removing a strain therefrom. In addition, in this case, the fermentation liquor may comprise the entire fermented product containing pantothenic acid produced by the microorganism or may be a fermented product from which impurities have been removed.

The fermentation liquor containing pantothenic acid may be obtained by culturing a microorganism producing pantothenic acid. The microorganism producing pantothenic acid may comprise both wild-type microorganisms and microorganisms that have undergone natural or artificial genetic modification, may be a microorganism of which specific mechanism is weakened or strengthened due to causes such as the insertion of an external gene or the enhancement or inactivation of endogenous gene activity, and may be a microorganism including a genetic modification of a target protein for producing pantothenic acid.

The microorganism producing pantothenic acid of the present application may be a microorganism which naturally has the ability to produce pantothenic acid or a microorganism in which the ability to produce pantothenic acid is conferred on a microorganism which does not have the ability to produce pantothenic acid, but is not limited thereto. Specifically, the microorganism producing pantothenic acid or the microorganism having the ability to produce pantothenic acid of the present application may be a microorganism in which some of genes in a pantothenic acid biosynthesis pathway are strengthened or weakened or some of the genes in a pantothenic acid degradation pathway are strengthened or weakened. The fact that the pantothenic acid production ability of the microorganism of the present application is "strengthened" or "increased" means that the ability to produce pantothenic acid of the microorganism of the present application is improved compared to a microorganism other than the microorganism of the present application, a parent strain, or an unmodified microorganism. As an example, the microorganism of the present application may be improved by about 1% or more, 2% or more, 5% or more, 10% or more, 100% or more, 200% or more, 500% or more, 1000% or more, 1100% or more, 1200% or more, or 1300% or more compared to the ability to produce pantothenic acid of other microorganisms, but is not limited thereto. The term "about" comprises all of ±0.5, ±0.4, ±0.3, ±0.2, ±0.1, etc., and comprises all values in a range equal to or similar to values following the term "about", but is not limited thereto.

The microorganism producing pantothenic acid may be an *Escherichia* sp. microorganism, a *Bacillus* sp. microorganism, a *Saccharomyces* sp. microorganism, or a *Corynebacterium* sp. microorganism. When the microorganism is the *Escherichia* sp. microorganism, the microorganism may be *Escherichia coli.* When the microorganism is the *Bacillus* sp. microorganism, the microorganism may be at least one selected from a group consisting of B. subtilis, B. vulgaris, B. cereus, etc. When the microorganism is the *Saccharomyces* sp. microorganism, the microorganism may be *Saccharomyces cerevisiae.* When the microorganism is the *Corynebacterium* sp. microorganism, the microorganism may be at least one selected from a group consisting of *Corynebacterium glutamicum, Corynebacterium erythrogenes, Corynebacterium crudilactis, Corynebacterium deserti, Corynebacterium efficiens, Corynebacterium callunae, Corynebacterium stationis, Corynebacterium singulare, Corynebacterium halotolerans, Corynebacterium striatum, Corynebacterium ammoniagenes, Corynebacterium pollutisoli, Corynebacterium imitans, Corynebacterium testudinoris, Corynebacterium crenatum,* and *Corynebacterium flavescens,* and for example, may be *Corynebacterium glutamicum,* but is not limited thereto.

The cultivation of the microorganism producing pantothenic acid may be performed according to an appropriate medium and culture conditions known in the art. Such a culture process may be easily adjusted and used by those skilled in the art according to the selected strain. Specifically, the cultivation may be a batch type, a continuous type, and a federation type, but is not limited thereto. The term "medium" in the present application refers to a material mixed with nutrients required for culturing the microorganism as a main ingredient, and supplies water essential for survival and growth, as well as nutrients and growth factors. Specifically, the medium and other culture conditions used for culturing the microorganism of the present application may be used without particular limitation, as long as the medium is used in the culture of normal microorganisms, but the microorganism of the present application may be cultured in a normal medium containing an appropriate carbon source, nitrogen source, phosphorus, inorganic compound, amino acid and/or vitamin under aerobic conditions while controlling temperature, pH, etc.

The removal of the bacterial cells (e.g., microorganisms or a part of microorganisms, etc.) from the fermentation liquor may be by a conventional method such as separation (e.g., centrifugation, etc.), filtration (e.g., use of a ceramic membrane filter, etc.).

The fermentation liquor which contains pantothenic acid and from which the bacterial cells have been removed may be passed through a cation exchange resin to remove impurities.

The cation exchange resin may remove cationic impurities. As the cation exchange resin, a commonly known cation exchange resin may be used, and for example, a strongly acidic cation exchange resin (SAC) or a weakly acidic cation exchange resin (WAC) may be used, but is not limited thereto. The cation exchange resin may be a gel type or a porous type, but is not limited thereto.

The cationic impurities may be alkali metal ions (e.g., sodium ions, potassium ions, etc.), alkaline earth metal ions (e.g., magnesium ions, calcium ions, etc.), or ammonium ions.

In this specification, the term "eluent" may refer to a fermentation liquor that has passed through a cation exchange resin or an anion exchange resin after the removal of the bacterial cells.

When the fermentation liquor from which the bacterial cells have been removed is excessively passed through the cation exchange resin, a large amount of cationic impurities that were not removed may be comprised in the eluent, and thus it is preferable to add the fermentation liquor to the cation exchange resin in an appropriate amount.

In order to add an appropriate amount of the fermentation liquor to the cation exchange resin, the step (a) may further comprise a step of checking the pH or Brix of the eluent which has passed through the cation exchange resin.

When the pH of the eluent is 3.0 or more, the cationic impurities, such as ammonium ions in the specific example using the fermentation liquor of the present application, may be excessively comprised in the eluent, so that an eluent having a pH of less than 3.0, less than 2.9, less than 2.8, less than 2.7, less than 2.6, less than 2.5, less than 2.4, less than 2.3, less than 2.2, less than 2.1, less than 2.0, less than 1.9 or less than 1.8; or 0.1 to 3, 0.1 to 2.9, 0.1 to 2.8, 0.1 to 2.7, 0.1 to 2.6, 0.1 to 2.5, 0.1 to 2.4, 0.1 to 2.3, 0.1 to 2.2, 0.1 to 2.1, 0.1 to 2, 0.1 to 1.9, 0.1 to 1.8, 0.25 to 3, 0.25 to 2.9, 0.25 to 2.8, 0.25 to 2.7, 0.25 to 2.6, 0.25 to 2.5, 0.25 to 2.4, 0.25 to 2.3, 0.25 to 2.2, 0.25 to 2.1, 0.25 to 2, 0.25 to 1.9, 0.25 to 1.8, 0.5 to 3, 0.5 to 2.9, 0.5 to 2.8, 0.5 to 2.7, 0.5 to 2.6, 0.5 to 2.5, 0.5 to 2.4, 0.5 to 2.3, 0.5 to 2.2, 0.5 to 2.1, 0.5 to 2, 0.5 to 1.9, 0.5 to 1.8, 0.75 to 3, 0.75 to 2.9, 0.75 to 2.8, 0.75 to 2.7, 0.75 to 2.6, 0.75 to 2.5, 0.75 to 2.4, 0.75 to 2.3, 0.75 to 2.2, 0.75 to 2.1, 0.75 to 2, 0.75 to 1.9, 0.75 to 1.8, 1 to 3, 1 to 2.9, 1 to 2.8, 1 to 2.7, 1 to 2.6, 1 to 2.5, 1 to 2.4, 1 to 2.3, 1 to 2.2, 1 to 2.1, 1 to 2, 1 to 1.9, or 1 to 1.8 may be obtained. In a specific example using the fermentation liquor of the present application, only an eluent having a pH of less than 1.8 may be obtained, but is not limited thereto.

The pH of the eluent passing through the cation exchange resin may be 3 or less, 2.5 or less, 2 or less, 1.8 or less, 0.1 to 3, 0.1 to 2.5, 0.1 to 2.25, 0.1 to 2, 0.1 to 1.8, 0.1 to 1.5, 0.25 to 3, 0.25 to 2.5, 0.25 to 2.25, 0.25 to 2, 0.25 to 1.8, 0.25 to 1.5, 0.5 to 3, 0.5 to 2.5, 0.5 to 2.25, 0.5 to 2, 0.5 to 1.8, 0.5 to 1.5, 0.75 to 3, 0.75 to 2.5, 0.75 to 2.25, 0.75 to 2, 0.75 to 1.8, 0.75 to 1.5, 1 to 3, 1 to 2.5, 1 to 2.25, 1 to 2, 1 to 1.8, 1 to 1.5, 1.25 to 3, 1.25 to 2.5, 1.25 to 2.25, 1.25 to 2, 1.5 to 3, 1.5 to 2.5, 1.5 to 2.25, 1.5 to 2, 1.75 to 3, 1.75 to 2.5, 1.75 to 2.25, or 1.75 to 2.

The Brix of the eluent may be lower than the Brix of the fermentation liquor from which the bacterial cells have been removed before passing through the cation exchange resin.

The method of producing the salt of pantothenic acid of the present application may not pass the fermentation liquor from which bacterial cells have been removed through an anion exchange resin. That is, the method of producing the salt of pantothenic acid of the present application may not comprise a step of using an anion exchange resin. There is an advantage that the yield of the salt of pantothenic acid can be increased, and the cost of processing basic chemicals used for regenerating the anion exchange resin can be reduced by not using the anion exchange resin.

The method of producing a salt of pantothenic acid of the present application may produce a salt of high-purity pantothenic acid even without using an anion exchange resin.

### Step (b)

The step (b) may be a step of adjusting the pH of the eluent obtained in the step (a).

The step of adjusting the pH may comprise a step of adding at least one selected from a group consisting of calcium hydroxide (Ca(OH)₂), calcium oxide (CaO), magnesium oxide (MgO), magnesium hydroxide (Mg(OH)₂), etc. to the eluent to adjust the pH of the eluent to 6 to 8, 6 to 7.8, 6 to 7.6, 6 to 7.5, 6 to 7.4, 6 to 7.2, 6 to 7, 6.5 to 8, 6.5 to 7.8, 6.5 to 7.6, 6.5 to 7.5, 6.5 to 7.4, 6.5 to 7.2, or 6.5 to 7, for example, 7.

The step (b) may further comprise a step of decolorizing the eluent with activated carbon. The salt of pantothenic acid may have a desirable color by performing the step of decolorizing the eluent with activated carbon.

The step (b) may further comprise a step of filtering after performing the decolorizing step.

The step (b) may further comprise a step of concentrating the eluent to obtain a concentrate. The step of concentrating may be performed after adjusting the pH of the eluent from which impurities have been removed in the step (a), or may be performed after adjusting the pH of the eluent from which impurities have been removed and decolorizing the eluent.

The step of concentrating may be performed using a conventional concentration method. The yield of the salt of pantothenic acid may be increased by performing the step of concentrating.

In an example, the step (b) may comprise a step of adjusting the pH of the eluent obtained in the step (a) and
a step of decolorizing the eluent of which pH has been adjusted with activated carbon.

In an example, the step (b) may comprise a step of adjusting the pH of the eluent obtained in the step (a),
a step of decolorizing the eluent of which pH has been adjusted with activated carbon, and
a step of concentrating the decolorized eluent to obtain a concentrate.

### Step (c)

The step (c) may be a step of adding an organic solvent to the pH-adjusted eluent obtained in the step (b).

The organic solvent may be a hydrophilic organic solvent, and may be at least one selected from a group consisting of a straight-chain or branched-chain alcohol having 1 to 4 carbon atoms (e.g., methanol, ethanol, isopropanol, etc.), acetone, etc.

The organic solvent may be used by mixing with water, and at this time, the concentration of the organic solvent [volume of organic solvent/(volume of water + organic solvent)] may be 90 to 100% (v/v), 95 to 100% (v/v), or 99 to 100% (v/v), but is not limited thereto.

The organic solvent may be added to the eluent until the final concentration is 50 to 100% (v/v), 50 to 99% (v/v), 50 to 95% (v/v), 50 to 90% (v/v), 50 to 85% (v/v), 50 to 82% (v/v), 50 to 80% (v/v), 55 to 100% (v/v), 55 to 99% (v/v), 55 to 95% (v/v), 55 to 90% (v/v), 55 to 85% (v/v), 55 to 82% (v/v), 55 to 80% (v/v), 60 to 100% (v/v), 60 to 99% (v/v), 60 to 95% (v/v), 60 to 90% (v/v), 60 to 85% (v/v), 60 to 82% (v/v), 60 to 80% (v/v), 65 to 100% (v/v), 65 to 99% (v/v), 65 to 95% (v/v), 65 to 90% (v/v), 65 to 85% (v/v), 65 to 82% (v/v), 65 to 80% (v/v), 70 to 100% (v/v), 70 to 99% (v/v), 70 to 95% (v/v), 70 to 90% (v/v), 70 to 85% (v/v), 70 to 82% (v/v), 70 to 80% (v/v), 75 to 100% (v/v), 75 to 99% (v/v), 75 to 95% (v/v), 75 to 90% (v/v), 75 to 85% (v/v), 75 to 82% (v/v), 75 to 80% (v/v), 80 to 100% (v/v), 80 to 99% (v/v), 80 to 95% (v/v), 80 to 90% (v/v), 80 to 85% (v/v), or 80 to 82% (v/v), but is not limited thereto.

By adding the organic solvent to the eluent, the solubility of pantothenic acid is reduced, so that a high recovery rate may be achieved in a crystallization step. As the concentration of the organic solvent in the eluent increases, the solubility of pantothenic acid decreases, so that an amount of pantothenic acid lost to a mother liquor (a solution remaining after filtering out solid (e.g., crystals of a salt of pantothenic acid) from a mixed solution of solid and liquid).

A concentration of the salt of pantothenic acid after adding the organic solvent to the eluent may be 10 to 500 g/L, 10 to 475 g/L, 10 to 450 g/L, 10 to 425 g/L, 10 to 400 g/L, 10 to 375 g/L, 10 to 350 g/L, 10 to 325 g/L, 10 to 300 g/L, 50 to 500 g/L, 50 to 475 g/L, 50 to 450 g/L, 50 to 425 g/L, 50 to 400 g/L, 50 to 375 g/L, 50 to 350 g/L, 50 to 325 g/L, 50 to 300 g/L, 100 to 500 g/L, 100 to 475 g/L, 100 to 450 g/L, 100 to 425 g/L, 100 to 400 g/L, 100 to 375 g/L, 100 to 350 g/L, 100 to 325 g/L, 100 to 300 g/L, 150 to 500 g/L, 150 to 475 g/L, 150 to 450 g/L, 150 to 425 g/L, 150 to 400 g/L, 150 to 375 g/L, 150 to 350 g/L, 150 to 325 g/L, 150 to 300 g/L, 200 to 500 g/L, 200 to 475 g/L, 200 to 450 g/L, 200 to 425 g/L, 200 to 400 g/L, 200 to 375 g/L, 200 to 350 g/L, 200 to 325 g/L, or 200 to 300 g/L, but is not limited thereto.

The step (c) may further comprise a step of obtaining crystals of a salt of pantothenic acid produced by adding the organic solvent.

The salt of pantothenic acid may be an alkaline earth metal salt of pantothenic acid (e.g., a calcium salt or a magnesium salt) and in an example, may be a calcium salt of pantothenic acid.

The step of obtaining the crystals of the salt of pantothenic acid (crystallization step) may be performed at a temperature of 25 °C or less.

Specifically, the crystallization step may be performed at a temperature of 0 to 25 °C, 0 to 22.5 °C, 0 to 20 °C, 0 to 17.5 °C, 0 to 15 °C, 0 to 12.5 °C, 0 to 10 °C, 0 to 7.5 °C, 0 to 5 °C, 0 to 2.5 °C, 1 to 25 °C, 1 to 22.5 °C, 1 to 20 °C, 1 to 17.5 °C, 1 to 15 °C, 1 to 12.5 °C, 1 to 10 °C, 1 to 7.5 °C, 1 to 5 °C, 1 to 2.5 °C, 2.5 to 25 °C, 2.5 to 22.5 °C, 2.5 to 20°C, 2.5 to 17.5 °C, 2.5 to 15°C, 2.5 to 12.5 °C , 2.5 to 10°C, 2.5 to 7.5 °C , 2.5 to 5 °C , 5 to 25 °C , 5 to 22.5 °C , 5 to 20 °C , 5 to 17.5 °C , 5 to 15 °C, 5 to 12.5 °C , 5 to 10 °C, or 5 to 7.5 °C .

When crystallizing at a temperature of 1 to 25 °C , it is possible to obtain prism-shaped pantothenic acid salt crystals (calcium pantothenate·4MeOH·1H₂O crystals) that are easy to separate, rather than fluffy needle-shaped D-pantothenic acid salt crystals (calcium D-pantothenate·MeOH), so that crystals may be separated with a higher recovery rate.

The step (c) may further comprise a step of introducing a crystallization seed.

When crystallizing the salt of pantothenic acid in the organic solvent, it takes about 8 days to obtain crystals. Therefore, a crystallization time may be reduced by adding a crystallization seed.

The crystallization seed may refer to small crystals of the same type added to obtain crystals from a supersaturated solution. The crystallization seed may be obtained by adding the organic solvent to the fermentation liquor. By adding the crystallization seed to the supersaturated solution, the added crystals become nuclei and grow into large crystals, and a speed of extracting the crystals may be accelerated and a size may be made uniform. In an example, the crystallization seed may be crystals of the salt of pantothenic acid, and specifically, it may be a calcium pantothenate·4MeOH·1H₂O crystals.

In an example, by introducing the crystallization seed in the fermentation liquor, the crystallization time may be reduced to 1 to 48 hours, 1 to 42 hours, 1 to 36 hours, 1 to 30 hours, 1 to 24 hours, 1 to 18 hours, 1 to 12 hours, 1 to 6 hours, 2 to 48 hours, 2 to 42 hours, 2 to 36 hours, 2 to 30 hours, 2 to 24 hours, 2 to 18 hours, 2 to 12 hours, 2 to 6 hours, 3 to 48 hours, 3 to 42 hours, 3 to 36 hours, 3 to 30 hours, 3 to 24 hours, 3 to 18 hours, 3 to 12 hours, 3 to 6 hours, 4 to 48 hours, 4 to 42 hours, 4 to 36 hours, 4 to 30 hours, 4 to 24 hours, 4 to 18 hours, 4 to 12 hours, 4 to 6 hours, 5 to 48 hours, 5 to 42 hours, 5 to 36 hours, 5 to 30 hours, 5 to 24 hours, 5 to 18 hours, 5 to 12 hours, 5 to 6 hours, 6 to 48 hours, 6 to 42 hours, 6 to 36 hours, 6 to 30 hours, 6 to 24 hours, 6 to 18 hours, or 6 to 12 hours.

As the salt of pantothenic acid is crystallized in the eluent, the concentration of the salt of pantothenic acid contained in the eluent decreases, and when the concentration of the salt of pantothenic acid contained in a residual liquor (mother liquor) excluding the crystals of the salt of pantothenic acid in the eluent is constant, the crystals of the salt of pantothenic acid may be separated and obtained.

The step (c) may further comprise a step of drying the obtained crystals.

The drying step may be performed one or more times, two or more times, or three or more times.

In an example, the drying step may be a step of drying for one day under room temperature and relative humidity of 20 to 25% (primary drying).

In another example, the drying step may comprise one or more drying steps (secondary drying) after the primary drying at a high temperature condition of 60 to 80 °C, 60 to 77.5 °C, 60 to 75 °C, 60 to 72.5 °C, 60 to 70 °C, 62.5 to 80 °C, 62.5 to 77.5 °C, 62.5 to 75 °C, 62.5 to 72.5 °C, 62.5 to 70 °C, 65 to 80 °C, 65 to 77.5 °C, 65 to 75 °C, 65 to 72.5 °C, 65 to 70 °C, 67.5 to 80 °C, 67.5 to 77.5 °C, 67.5 to 75 °C, 67.5 to 72.5 °C, or 67.5 to 70 °C.

Since a melting point of the crystals (e.g., calcium pantothenate·4MeOH·1H₂O) is 55 to 60 °C , there is a problem that the crystals are melted and it is difficult to maintain the crystals form when drying the crystals directly under the high temperature condition. However, as a result of performing the primary drying step, the organic solvent in the crystals is volatilized, thereby achieving an effect of increasing the melting point of the crystals. Therefore, the obtained crystals may be primarily dried and then secondarily dried to obtain the crystals of the salt of pantothenic acid more stably.

In an example, the step (c) may comprise a step of adding an organic solvent to the concentrate obtained in the step (b), and
a step of obtaining crystals of a salt of pantothenic acid produced by adding the organic solvent.

In an example, the step (c) may comprise a step of adding an organic solvent to the concentrate obtained in the step (b),
a step of obtaining crystals of a salt of pantothenic acid produced by adding the organic solvent, and
a step of drying the obtained crystals.

In an example, the step (c) may comprise a step of adding an organic solvent to the concentrate obtained in the step (b),
a step of introducing a crystallization seed,
a step of obtaining crystals of a salt of pantothenic acid produced by adding the organic solvent and the crystallization seed, and
a step of drying the obtained crystals.

### Property of crystals of salt of pantothenic acid

A salt of pantothenic acid produced by the producing method of the present application may have a purity of 98% or more, 98.1% or more, 98.2% or more, 98.3% or more, 98.4% or more, 98.5% or more, 98.6% or more, 98.7% or more, 98.9% or more, 99% or more, 99.1% or more, 99.2% or more, 99.3% or more, 99.4% or more, 99.5% or more, 99.6% or more, 99.7% or more, 99.8% or more, or 99.9% or more.

When the salt of pantothenic acid is produced by the producing method of the present application, a recovery rate of the salt of pantothenic acid may be 70% or more, 75% or more, 80% or more, or 85% or more.

The purity of the salt of pantothenic acid produced by the producing method of the present application may be 0.1%p or more, 0.2%p or more, 0.3%p or more, 0.4%p or more, 0.5%p or more, 0.6%p or more, 0.7%p or more, 0.8%p or more, 0.9%p or more, 1.0%p or more, 1.1%p or more, 1.2%p or more, 1.3%p or more, or 1.4%p or more higher than a purity of a salt of pantothenic acid produced by a producing method including a step of using an anion exchange resin and/or not including a step of using a crystallization seed, but is not limited thereto.

The recovery rate of the salt of pantothenic acid produced by the producing method of the present application may be 1%p or more, 2%p or more, 3%p or more, 4%p or more, 5%p or more, 6%p or more, 7%p or more, 8%p or more, 9%p or more, 10%p or more, 12%p or more, 14%p or more, 15%p or more, 16%p or more, 18%p or more, 19%p or more, 20%p or more, 25%p or more, 26%p or more, 29%p or more, or 30%p or more higher than a recovery rate of the salt of pantothenic acid produced by the producing method including the step of using the anion exchange resin and/or not including the step of using the crystallization seed, but is not limited thereto.

Another aspect provides crystal of a salt of pantothenic acid obtained by a method comprising the steps of:
(a') passing a fermentation liquor from which bacterial cells have been removed through a cation exchange resin;
(b') adjusting the pH to 6 to 8; and
(c') adding an organic solvent.

Fermentation liquor, the cation exchange resin, the organic solvent, the salt of pantothenic acid or the crystals thereof are as described above.

### ADVANTAGEOUS EFFECTS

The method of producing a salt of pantothenic acid of the present application can prevent losses that may occur in a process using an anion exchange resin by simplifying an existing process using a cation exchange resin and the anion exchange resin, and can achieve an excellent effect of obtaining a salt of pantothenic acid with high purity even without using the anion exchange resin.

### BRIEF DESCRIPTION OF THE DRAWINGS

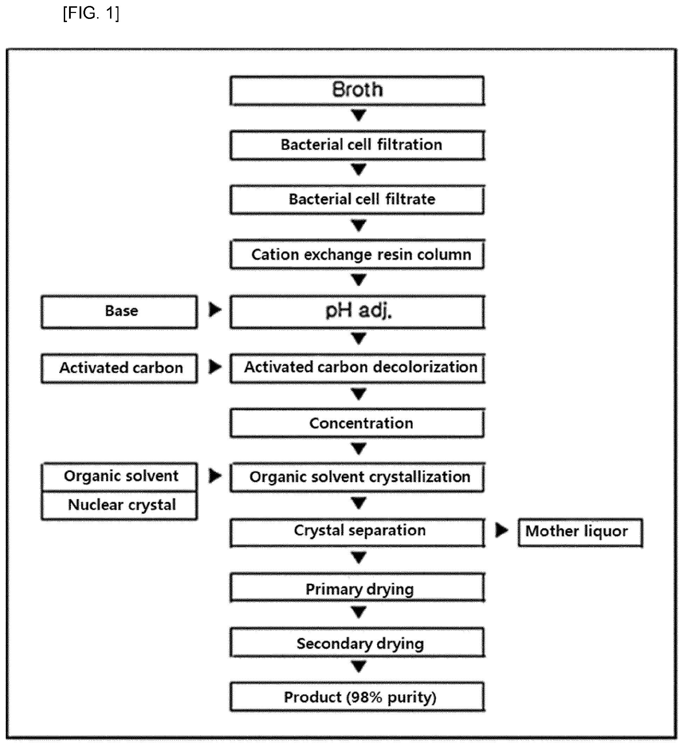

FIG. 1 is a view of a purification process for a salt of pantothenic acid.

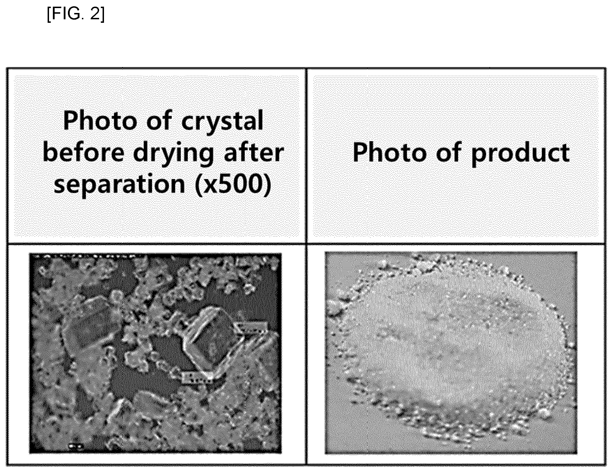

FIG. 2 is a photograph of crystals form before drying and a product after drying after the crystallization process according to the present application is completed.

### MODE FOR INVENTION

Hereinafter, the present invention will be described more specifically with reference to the following examples. However, these are only intended to illustrate the present invention, and the scope of the present invention is not limited by these examples.

### Experimental Example 1-1. Crystallization process using eluent that passed through cation exchange resin (1)

Bacterial cells were removed from a fermentation liquor (pantothenic acid concentration 46 g/L, specific gravity 1.025, solid content 9.8% (w/w), bacterial cell PCV (packed cell volume) 9% (w/w)) of *Corynebacterium glutamicum* strain containing D-pantothenic acid using a ceramic membrane filter. The fermentation liquor from which the bacterial cells were removed was passed through a cation exchange resin tower to obtain an eluent with a pH of less than 1.8 from which impurities were removed, the pH was adjusted to 7 with calcium hydroxide (Ca(OH)₂), and activated carbon was added to be decolorized and then filtered.

The filtrate containing 58.8 g of calcium D-pantothenate was concentrated to 542 g/L of calcium D-pantothenate, and then methanol was added to a final concentration of 70%. 0.6 g of crystallization seed (calcium salt of D-pantothenate; prism-shaped calcium D-pantothenate·4MeOH·1H₂O) was added thereto, and after stirring at 10 °C for 24 hours, 44.6 g of D-pantothenate calcium salt crystals were separated.

The separated crystals were dried at room temperature and a relative humidity of 25% for one day, and then further dried in an oven at 70 °C to finally obtain 44.6 g of calcium D-pantothenate. In this case, the crystal purity was 99.7%, and the recovery rate was 75.8%.

### Experimental Example 1-2. Crystallization process using eluent passing through cation exchange resin tower (2)

Bacterial cells were removed from a fermentation liquor (pantothenic acid concentration 46 g/L, specific gravity 1.025, solid content 9.8% (w/w), bacterial cell PCV 9% (w/w)) of *Corynebacterium glutamicum* strain containing D-pantothenic acid using a ceramic membrane filter. The fermentation liquor from which the bacterial cells were removed was passed through a cation exchange resin tower to obtain an eluent with a pH of less than 1.8 from which impurities were removed, the pH was adjusted to 7 with calcium hydroxide (Ca(OH)₂), and activated carbon was added to be decolorized and then filtered.

The filtrate containing 71.2 g of calcium D-pantothenate was concentrated to 547 g/L of calcium D-pantothenate, and then methanol was added to a final concentration of 70% (v/v). 0.71 g of crystallization seed (calcium salt of D-pantothenate; prism-shaped calcium D-pantothenate·4MeOH·1H₂O) was added thereto, and after stirring at 10 °C for 24 hours, 53.75 g of D-pantothenate calcium salt crystals were separated.

The separated crystals were dried at room temperature and a relative humidity of 25% for one day, and then further dried in an oven at 70 °C to finally obtain 53.75 g of calcium D-pantothenate. In this case, the crystal purity was 101.4%, and the recovery rate was 75.5%.

### Experimental Example 1-3. Crystallization process using eluent passing through cation exchange resin tower (3)

Bacterial cells were removed from a fermentation liquor (pantothenic acid concentration 46 g/L, specific gravity 1.025, solid content 9.8% (w/w), bacterial cell PCV 9% (w/w)) of *Corynebacterium glutamicum* strain containing D-pantothenic acid using a ceramic membrane filter. The fermentation liquor from which the bacterial cells were removed was passed through a cation exchange resin tower to obtain an eluent with a pH of less than 1.8 from which impurities were removed, the pH was adjusted to 7 with calcium hydroxide (Ca(OH)₂), and activated carbon was added to be decolorized and then filtered.

The filtrate containing 58.8 g of calcium D-pantothenate was concentrated to 620 g/L of calcium D-pantothenate, and then methanol was added to a final concentration of 82% (v/v).

0.6 g of crystallization seed (calcium salt of D-pantothenate; prism-shaped calcium D-pantothenate·4MeOH·1H₂O) was added thereto, and after stirring at 10 °C for 24 hours, 48.6 g of D-pantothenate calcium salt crystals were separated.

The separated crystals were dried at room temperature and a relative humidity of 25% for one day, and then further dried in an oven at 70 °C to finally obtain 48.6 g of calcium D-pantothenate. In this case, the crystal purity was 98.9% and the recovery rate was 82.6%.

The results of the crystallization process according to Experimental Examples 1-1 to 1-3 are summarized in Table 1 below.

**[Table 1]**

| Crystallization results according to eluent conditions during crystallization | | | | |
|---|---|---|---|---|
| | Experiment No. | Experimental Example 1-1 | Experimental Example 1-2 | Experimental Example 1-3 |
| Eluent | Concentration of calcium D-pantothenate in eluent (g/L) | 203.8 | 222.8 | 280V.0 |
| | Content of MeOH (%(v/v)) | 70.0 | 70.0 | 82.5 |
| | Purity of calcium D-pantothenate in eluent (%) | 73.4 | 73.4 | 69.7 |
| Crystalliz ation result | Crystal purity (%) | 99.7 | 101.4 | 98.9 |
| | Recovery rate of crystal (%) | 75.8 | 75.5 | 82.6 |

The purity of calcium D-pantothenate in the eluent was calculated as (weight of calcium pantothenate) X 100 / (weight of calcium pantothenate and other impurities) in the eluent. The purity of the crystals was calculated as (weight of pantothenic acid in 1 g of the obtained crystals) X 100 / (weight of pantothenic acid in 1 g of calcium pantothenate with 100% purity). The crystal recovery rate was calculated as (weight of calcium pantothenate crystals) X 100 / (weight of pantothenic acid in the fermentation liquor).

### Comparative Example 1. Crystallization process using eluents passed through cation and anion exchange resin towers

Bacterial cells were removed from a fermentation liquor (pantothenic acid concentration 46 g/L, specific gravity 1.025, solid content 9.8%, bacterial cell PCV 9%) of *Corynebacterium glutamicum* strain containing D-pantothenic acid using a ceramic membrane filter. The fermentation liquor from which the bacterial cells were removed was passed through a cation exchange resin tower to obtain an eluent with a pH of less than 1.8, and was sequentially passed through an anion exchange resin tower to obtain an eluent from which impurities were removed, and then the pH was adjusted to pH 7 with calcium hydroxide (Ca(OH)₂). After that, activated carbon was added to be decolorized and then filtered.

The filtrate containing 41 g of calcium D-pantothenate was concentrated to 570 g/L of calcium D-pantothenate, and then methanol was added to the final concentration of 70% (v/v). 0.41 g of crystallization seed (calcium salt of D-pantothenate; prism-shaped calcium D-pantothenate·4MeOH·1H₂O) was added thereto, and after stirring at 10 °C for 24 hours, 33.75 g of D-pantothenate calcium salt crystals were separated.

The separated crystals were dried at room temperature and a relative humidity of 25% for one day, and then further dried in an oven at 70 °C to finally obtain 33.75 g of calcium D-pantothenate. In this case, the crystal purity was 98.5% and the recovery rate was 53.8%.

### Comparative Example 2. Crystallization process without crystallization seed (1)

Bacterial cells were removed from a fermentation liquor (pantothenic acid concentration 46 g/L, specific gravity 1.025, solid content 9.8%, bacterial cell PCV 9%) of *Corynebacterium glutamicum* strain containing D-pantothenic acid using a ceramic membrane filter. The fermentation liquor from which the bacterial cells were removed was passed through a cation exchange resin tower to obtain an eluent with a pH of less than 1.8 from which impurities were removed, and then the pH was adjusted to pH 7 with calcium hydroxide (Ca(OH)₂). After that, activated carbon was added to be decolorized and then filtered.

The filtrate containing 37 g of calcium D-pantothenate was concentrated to 620 g/L of calcium D-pantothenate, and then methanol was added to a final concentration of 70% (v/v).

Thereafter, after stirring at 10 °C for 8 days without adding a crystallization seed, crystals were separated, and 27.3 g of D-pantothenate calcium salt crystals were separated. When no crystallization seed was added, no crystals were formed until 8 days.

The separated crystals were dried for one day at room temperature and relative humidity of 25%, and then further dried in an oven at 70 °C to finally obtain 27.3 g of calcium D-pantothenate. In this case, the crystal purity was 99.1%, and the crystal recovery rate was 74%.

### Comparative Example 3. Crystallization process without crystallization seed (2)

Bacterial cells were removed from a fermentation liquor (pantothenic acid concentration 46 g/L, specific gravity 1.025, solid content 9.8%, bacterial cell PCV 9%) of *Corynebacterium glutamicum* strain containing D-pantothenic acid using a ceramic membrane filter. The fermentation liquor from which the bacterial cells were removed was passed through a cation exchange resin tower to obtain an eluent with a pH of less than 1.8, and was sequentially passed through an anion exchange resin tower to obtain an eluent from which impurities were removed, and then the pH was adjusted to pH 7 with calcium hydroxide (Ca(OH)₂). After that, activated carbon was added to be decolorized and then filtered.

The filtrate containing 41 g of calcium D-pantothenate was concentrated to 570 g/L of calcium D-pantothenate, and then methanol was added to the final concentration of 70% (v/v).

Thereafter, after stirring at 10 °C for 8 days without adding a crystallization seed, crystals were separated, and 30.75 g of pantothenic acid calcium salt crystals were separated. When no crystallization seed was added, no crystals were formed until 8 days.

The separated crystals were dried at room temperature and relative humidity of 25% for one day and then further dried in an oven at 70 °C to finally obtain 30.75 g of calcium D-pantothenate. In this case, the crystal purity was 98.5%, and the crystal recovery rate was 56.3%.

From the description, those skilled in the art will understand that the present invention may be implemented in other specific forms without changing the technical idea or essential features thereof. In this regard, it should be understood that embodiments described above are exemplary in all respects and not restrictive. The scope of the present invention should be interpreted as including all changes or modifications derived from the meaning and scope of the patent claims described below rather than the detailed description and their equivalent concepts within the scope of the present invention.

## Claims

1. A method of producing a salt of pantothenic acid, the method comprising steps of:
(a) passing a fermentation liquor which contains pantothenic acid and from which bacterial cell has been removed through a cation exchange resin to obtain an eluent from which impurity has been removed;
(b) adjusting pH of the eluent obtained in the step (a); and
(c) adding an organic solvent to the pH-adjusted eluent obtained in the step (b).

2. The method of claim 1, wherein the step (b) is a step of adjusting **pH** of the eluent to 6 to 8.

3. The method of claim 1, wherein the step (b) is a step of adjusting the **pH of** the eluent by using at least one selected from a group consisting of calcium hydroxide, calcium oxide, magnesium hydroxide, and magnesium oxide.

4. The method of claim 1, wherein the step (b) further comprises a step of decolorizing the eluent with activated carbon.

5. The method of claim 1, wherein the step (b) further comprises a step of concentrating the fermentation liquor.

6. The method of claim 1, wherein the organic solvent in the step (c) is a straight-chain or branched-chain alcohol having 1 to 4 carbon atoms, or acetone.

7. The method of claim 1, wherein the step (c) further comprises a step of obtaining crystal of a salt of pantothenic acid produced by adding an organic solvent.

8. The method of claim 1, wherein the step (c) further comprises a step of introducing a crystallization seed.

9. The method of claim 7, wherein the step of obtaining crystal of the salt of pantothenic acid in step (c) is to crystallize at 1 °C to 25 °C.

10. The method of claim 7, wherein the step (c) further comprises a step of drying the obtained crystal.

11. The method of any one of claims 1 to 10, wherein the method does not comprise a step of using an anion exchange resin.

12. Crystal of a salt of pantothenic acid prepared by the method of any one of claims 1 to 10.
